# EUROPEAN PATENT APPLICATION

(11) **EP 2 392 275 A2**
(43) Date of publication of application: **07.12.2011**
(21) Application number: 11250581.3
(22) Date of filing: 06.06.2011
(51) Int. Cl.: A61B 17/34, A61B 19/00

(54) **Pressure feedback access ports for minimally invasive surgery**

(30) Priority: 07.06.2010 US 351999 P; 27.04.2011 US 95411
(71) Applicant: Tyco Healthcare Group LP, New Haven, CT 06511 (US)
(72) Inventor: Craft, Brandon Wesley, Reisterstown, MD 21136 (US); Haig, Fiona M, Cambridge CB24 9JG (GB); Collier, Nicholas John, Cambridge CB25 9JG (GB); Clark, Charlotte Adele, Cambridge CB4 1QB (GB); O'Prey, Cormac, Bishops Stortford Hertfordshire CM23 4HH (GB)
(74) Representative: Soames, Candida Jane

(57) **Abstract**

A surgical access port configured and dimensioned for positioning within an opening in tissue for providing access to an internal surgical site includes a body portion defining a passageway therethrough. The body portion includes an outer surface. A pressure feedback indicator is disposed on at least a portion of the outer surface of the body portion. The pressure feedback indicator is configured for providing a visual indication to a user where an applied pressure is greater than a predetermined threshold.

## Description

### BACKGROUND

This application claims priority from provisional application serial no. 61/351,999, filed June 7, 2010, the entire contents of which are incorporated herein by reference.

### 1. Technical Field

The present disclosure relates generally to devices and techniques for performing minimally invasive surgical procedures, and more particularly, to access ports that facilitate access to an internal surgical site.

### 2. Background of the Related Art

In an effort to reduce trauma and recovery time, many surgical procedures are performed through small openings in the skin, such as an incision or a natural body orifice. For example, these procedures include laparoscopic procedures, which are generally performed within the confines of a patient's abdomen, and thoracic procedures, which are generally performed within a patient's chest cavity.

Specific surgical instruments have been developed for use during such minimally invasive surgical procedures. These surgical instruments typically include an elongated shaft with operative structure positioned at a distal end thereof, such as graspers, clip appliers, specimen retrieval bags, etc.

During minimally invasive procedures, the clinician creates an opening in the patient's body wall, oftentimes by using an obturator or trocar, and thereafter positions an access assembly within the opening. The access assembly includes a passageway extending therethrough to receive one or more of the above-mentioned surgical instruments for positioning within the internal work site, e.g. the body cavity.

During minimally invasive thoracic procedures, an access assembly is generally inserted into a space located between the patient's adjacent ribs that is known as the intercostal space, and then surgical instruments can be inserted into the internal work site through the passageway in the access assembly.

During these procedures, firm, reliable placement of the access assembly is desirable to allow the access assembly to withstand forces that are applied during manipulation of the instrument(s) inserted therethrough. However, reducing patient trauma during the procedure, discomfort during recovery, and the overall recovery time remain issues of importance. Thus, there exists a need for thoracic access ports which minimize tissue trauma, discomfort and post operative patient pain.

### SUMMARY

In accordance with one aspect of the present disclosure, a surgical access port configured and dimensioned for positioning within an opening in tissue for providing access to an internal surgical site is provided. The surgical access port includes a body portion defining a passageway therethrough and has an outer surface. A pressure feedback indicator is on at least a portion of the outer surface of the body portion. The pressure feedback indicator is configured for providing a visual indication to a user where an applied pressure is greater than a predetermined threshold.

The pressure feedback indicator may include in some embodiments a pressure sensitive paint coating at least a portion of the outer surface of the access port, a pressure sensitive gel encapsulated in a casing disposed about at least a portion of the outer surface of the access port, and/or a pressure sensitive material that forms at least part the outer surface of the access port.

The visual indication may include in some embodiments a change in color of the pressure feedback indicator at the location (or locations) on the outer surface of the access port where the applied pressure is greater than the predetermined threshold. In some embodiments, the pressure feedback indicator may be configured to change color at the location on the outer surface from an initial color to an "indication" color. The initial color may correspond to an applied pressure that is less than the predetermined threshold, while the "indication" color may correspond to an applied pressure that is greater than the predetermined threshold, thus providing a visual indication as to the location of the applied pressure which exceeds the predetermined threshold.

In some embodiments, the pressure feedback indicator may be further configured to provide a visual indication as to the relative amount of applied pressure at the location (or locations) on the outer surface of the access port. More specifically, the pressure feedback indicator may be configured to change color at the location on the outer peripheral surface where the applied pressure is greater than the predetermined threshold through a spectrum of colors, or shades of colors, between the initial color and a final color. The initial color may correspond to applied pressure(s) equal to or less than the predetermined threshold, while the final color may correspond to a specific applied pressure, or pressure range, that is greater than the predetermined threshold. Color changes in between may be provided according to specific pressures, or pressure ranges, to further define the relative applied pressure at a specific location on the outer surface of the access port for visual feedback as to the relative amount of pressure being applied.

The body portion of the access port can have a first region external of the opening in tissue and a second region internal of the opening in tissue, wherein the pressure feedback indicator is on the first region of the body portion. Alternatively, or in addition, the pressure feedback indicator can be on the second region of the body portion. In an embodiment, the visual indication includes a change to a first indication color in the first region and a change to a second indication color in the second region.

In another aspect, a surgical access port is provided that is configured and dimensioned for positioning within an opening in tissue for providing access to an internal surgical site, the surgical access port comprising a body portion defining a passageway therethrough. The body portion extends through the tissue opening and includes a pressure feedback indicator providing an indication to the user of a change in pressure on the body portion. The pressure feedback indicator can be at a proximal region and/or a distal region of the body portion and can be responsive to pressure applied by a medical instrument extending through the passageway. The indication in some embodiments is a visual indication including a change to a first indication color in a first region and a change to a second indication color in a second region.

In accordance with another aspect of the present disclosure, a method of facilitating access to an internal surgical site beneath a patient's tissue is provided. The method includes forming an opening in the patient's tissue and advancing an access port through the opening. The access port defines a passageway therethrough and includes a pressure feedback indicator on at least a portion of the outer surface thereof. The method further includes beginning a surgical procedure within the internal surgical site via the passageway defined within the access port, observing the visual indication (or indications) as to the location (or locations) on the outer surface where the applied pressure is greater than the pre-determined threshold and modifying the surgical procedure according to the observed visual indication(s) to reduce the applied pressure to a pressure less than the pre-determined threshold. In some embodiments, the visual indication provided by the pressure feedback indicator includes a change of color on the outer surface.

### BRIEF DESCRIPTION OF THE DRAWINGS

Various exemplary embodiments of the present disclosure are described hereinbelow with reference to the drawings, wherein:

FIG. 1 is a front view illustrating a patient's skeletal structure with one embodiment of the presently disclosed surgical access port positioned between the intercostal space defined between the patient's adjacent ribs in accordance with the present disclosure;

FIG. 2 is a side, perspective view of the access port of Fig. 1 positioned within the intercostal space with a surgical instrument inserted therethrough;

FIG. 3 is a front, perspective view of an alternative embodiment of the access port of the present disclosure;

FIG. 4 is a side, perspective view illustrating another embodiment of the access port of the present disclosure positioned within the intercostal space;

FIG. 5 is a side, perspective view of yet another embodiment of the access port of the present disclosure;

FIG. 6 is a cross-sectional view illustrating another embodiment of the access port of the present disclosure positioned within the intercostal space;

FIG. 7 is a front, perspective view of still another embodiment of the access port of the present disclosure;

FIG. 8 is a front, perspective view of yet another embodiment of the access port of the present disclosure;

FIG. 9 is a cross-sectional view of the access port of FIG. 8 with a hand of a surgeon inserted therethrough; and

FIG. 10 is a schematic illustration of an access port providing visual feedback in response to pressure being applied to the access port.

### DETAILED DESCRIPTION OF THE EMBODIMENTS

Various embodiments of the presently disclosed access port, and methods of using the same, will now be described in detail with reference to the drawings wherein like references numerals identify similar or identical elements. In the drawings, and in the following description, the term "proximal" should be understood as referring to the end of the access port, or component thereof, that is closer to the clinician during proper use, while the term "distal" should be understood as referring to the end that is farther from the clinician, as is traditional and conventional in the art.

As will be described in greater detail hereinbelow, the present disclosure relates to access ports capable of providing a surgeon with feedback, e.g., a visual indication, such as a color change, as to the location (or locations) and/or the relative amount (or amounts) of pressure being applied to the access port. Although several surgical access ports are described hereinbelow, it is envisioned that the presently disclosed pressure feedback indicator feature may be provided for use with any suitable surgical access ports. Further, although specific pressure feedback features may be described in conjunction with one or more of the access ports described herein, it is envisioned that the other pressure feedback features disclosed herein, or any other suitable pressure feedback features, may be provided for use with the access ports of the present disclosure.

More specifically, the presently disclosed pressure-feedback access ports alert a surgeon as to the location (or locations) and/or relative amount (or amounts) of pressure applied to the access port and, thus, alert the surgeon as to the location(s) and/or relative amount(s) of pressure applied to the patient's tissue during the course of a surgical procedure, e.g., during insertion, removal, and/or manipulation of surgical instrument(s) through the access ports. As a result, the pressure-feedback feature allows the surgeon to adjust the positioning of the surgical instruments within the access port and/or to modify the surgical technique used in order to alleviate the influence of such pressures upon the patient's tissues, and consequently, to reduce patient trauma, discomfort following the procedure, and recovery time.

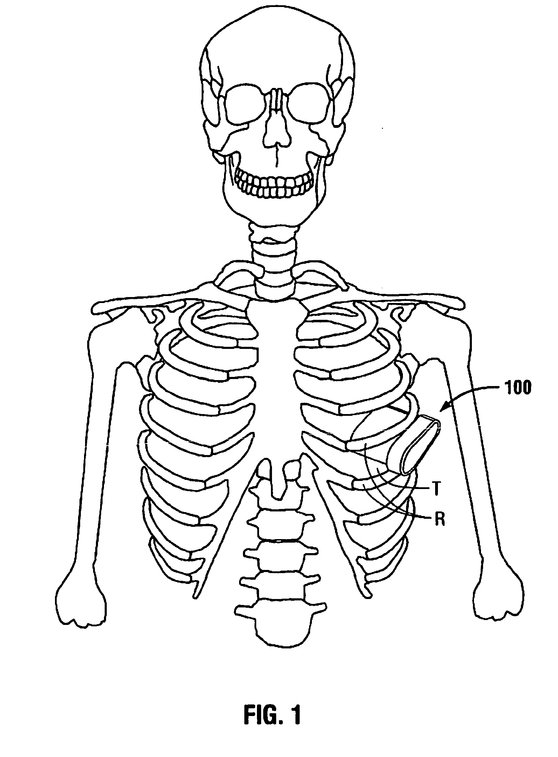

FIGS. 1-2 illustrate one embodiment of the presently disclosed surgical access port, which is identified by the reference character 100, in use during the course of a minimally invasive thoracic surgical procedure. As such, in the embodiment of the access port 100 seen in FIGS. 1-2, the access port 100 is depicted as a thoracic port that is configured and dimensioned for insertion into the intercostal space located between the adjacent ribs "R" of a patient in order to allow for the insertion and manipulation of one or more surgical instruments within the thoracic cavity "T." It is envisioned that the access port 100 may be formed from any suitable biocompatible material of a strength suitable for the purpose described herein, including, but not being limited to, polymeric materials.

The access port 100 is configured and dimensioned to extend into the thoracic cavity "T" through the intercostal space, and includes a hollow body portion 102. The body portion 102 includes a proximal portion 104 with an open proximal end 106, a distal portion 108 with an open distal end 110, and defines an internal space, or passageway 112 , extending from open proximal end 104 and to open distal end 110, that is configured and dimensioned to receive one or more surgical instruments "I."

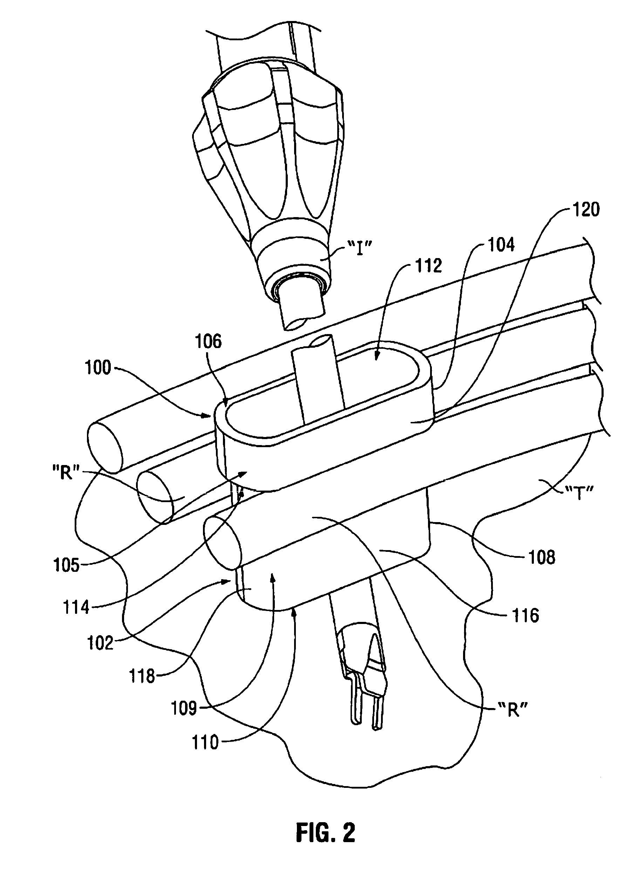

As best seen in FIG. 2, in one embodiment of the access port 100, the proximal portion 104 of the body portion 102 defines a greater transverse dimension than the distal portion 108. The larger proximal portion 104 facilitates manual engagement, e.g., gripping, by the clinician, and defines a flange, or buffer, 114 that is configured and dimensioned for abutment with the patient's tissue, e.g., the patient's ribs "R" during distal advancement of the access port 100 through the intercostal space. Contact between the flange 114 and the patient's tissue prevents the access port 100 from passing entirely into the thoracic cavity "T."

The body portion 102 of the access port 100, as illustrated in FIGS. 1-2, includes a pair of planar first sidewalls 116 and a pair of arcuate second sidewalls 118, such that body portion 102 generally defines an elongated, substantially oval cross-sectional configuration. The substantially planar configuration of the pair of first sidewalls 116 maximizes the surface area available for contact with the patient's tissue. The substantially oval cross-sectional configuration also better conforms to the elongated incision.

In an alternative embodiment of the access port 100, it is envisioned that both the pair of first sidewalls 116 and the pair of second sidewalls 118 may be substantially planar in configuration such that the cross-sectional configuration of the body portion 102 is substantially rectangular.

The specific configuration and dimensions of the access port 100 may be varied in alternative embodiments of the present disclosure based on such factors as the anatomy of the patient to be treated, and the surgical instruments to be used in conjunction therewith. As such, it is further envisioned that both pairs of sidewalls 116, 118 may include arcuate profiles in order to further facilitate spreading of the tissue, as well as maximization of the internal space 112 defined within the body portion 102.

As mentioned above, it is envisioned in some applications that the body portion 102 may be dimensioned such that the tissue adjacent the patient's ribs "R" are spread apart during distal advancement of the access port 100. If necessary, in order to further spread the tissue adjacent the patient's ribs "R", or in some instances the ribs, the access port 100 may be rotated. In order to maintain displacement of the tissue in the manner described, and/or in order to maintain passageway 112 open to facilitate the insertion and manipulation of the surgical instrument(s) "I" therethrough, it is envisioned that the material comprising the access port 100 may be of sufficient rigidity to resist excessive bending under the conditions normally encountered during such a surgical procedure.

Referring now to FIG. 2 in particular, the access port 100 is shown inserted into the thoracic cavity "T" between adjacent ribs "R" with a surgical instrument "I" inserted therethrough. The surgical instrument "I" may be any surgical instrument that is configured and dimensioned to pass through the body portion 102 of the access port 100, and adapted to perform a surgical, diagnostic, or other desired procedure. For example, suitable surgical instruments "I" may include endoscopic apparatus, which perform a variety of functions such as specimen retrieval and the application of surgical clips or other such fasteners to, and/or the cutting of, body tissue.

With continued reference to FIG. 2, an outer peripheral surface 105 of the proximal portion 104 of the body 102 of access port 100 and/or an outer peripheral surface 109 of the distal portion 108 of the body 102 of access port 100 (or portions thereof) may include a pressure feedback indicator or member 120 disposed thereon. In the embodiment of FIG. 2, the pressure feedback member 120 is disposed on the proximal portion 104. More particularly, in this embodiment, the outer peripheral surface 105 of proximal portion 104 of access port 100 is coated with a biocompatible, pressure sensitive paint. The pressure sensitive paint 120, as will be described in greater detail below, is configured for providing visual feedback as to the location and/or relative amount of pressure applied to the outer peripheral surface 105 of the proximal portion 104 of access port 100.

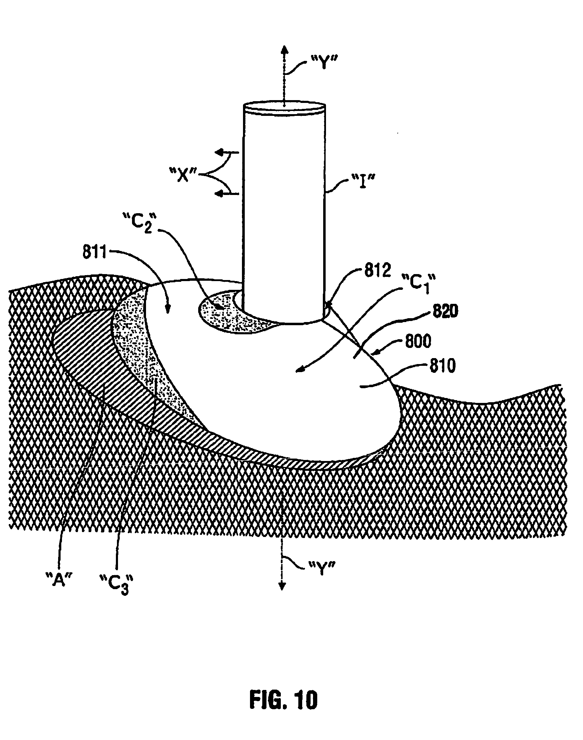

Although the pressure feedback indicator 120 is shown as a pressure sensitive paint, it is envisioned that the outer peripheral surface 105 of proximal portion 104 and/or the outer peripheral surface 109 of distal portion 108 of the body portion 102 of access port 100, or portions thereof, may include other pressure feedback indicators, such as a pressure sensitive gel encapsulated in a transparent casing. The pressure feedback indicator can be applied or positioned on a region, or alternatively on the entire region, of peripheral surface 105 and/or peripheral surface 109. Alternatively, the outer peripheral surfaces 105, 109 of the proximal and distal portions 104, 108, respectively, of the access port 100 may be formed partially from a pressure sensitive material, or alternatively, formed entirely from a pressure sensitive material. The functionality of the pressure feedback indicator 120, e.g., pressure sensitive paint, will be discussed in greater detail below with reference to FIG. 10.

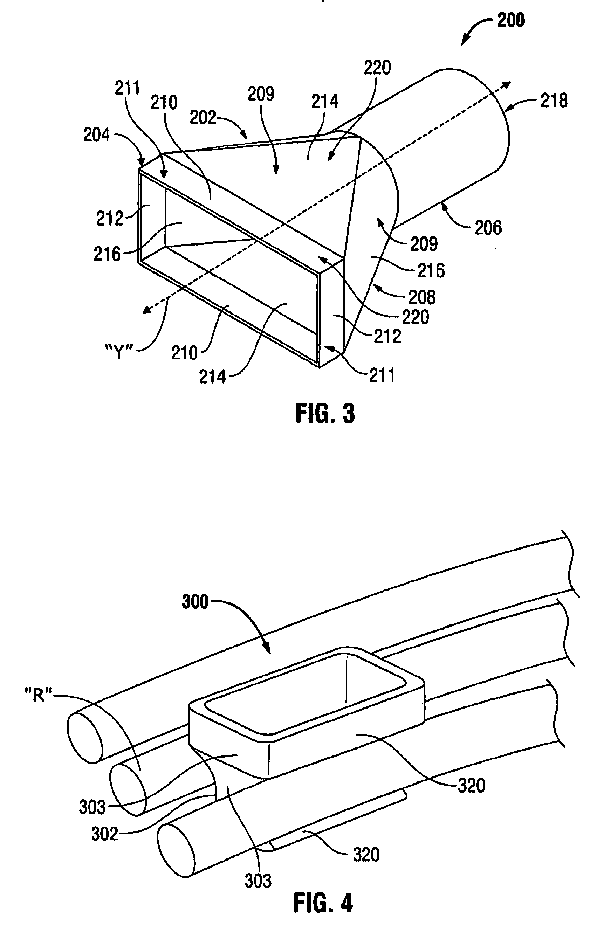

With reference now to FIGS. 3-6, alternative embodiments of the presently disclosed access ports will be discussed.

FIG. 3 illustrates an embodiment of the presently disclosed access port that is identified by the reference character 200. The access port 200 includes a body portion 202 having a proximal portion 204, a distal portion 206, and an intermediate portion 208 extending therebetween along a longitudinal axis "Y."

The proximal portion 204 includes respective first and second pairs of sidewalls 210, 212. Each pair of sidewalls extend in substantially parallel relation such that the proximal portion 204 defines a substantially rectangular configuration. However, it should be appreciated that alternative configurations for the proximal portion 204, such as substantially oval, are within the scope of the present disclosure. Thus, for example, one or more of the sidewalls could be arcuate in configuration. The configuration and dimensions of the proximal portion 204 facilitate the insertion into, and the removal of the surgical instrument(s) "I" (FIG. 2) from, the access port 200.

The intermediate portion 208 is integral with, or connected to, the proximal portion 204 and extends distally therefrom. The intermediate portion 208 includes respective first and second pairs of sidewalls 214, 216 extending from and corresponding to the sidewalls 210, 212, respectively, of the proximal portion 204. The second pair of sidewalls 216 taper inwardly towards the longitudinal axis "Y." The inward taper of the second pair of sidewalls 216 guides the surgical instrument(s) "I" upon insertion into the access port 200 to facilitate passage into the thoracic cavity "T" (FIGS. 1-2). The taper provides a surface that is configured and dimensioned for abutment with the patient's tissue to prevent the surgical access port 200 from passing entirely into the internal work site as the cross-sectional dimension of the access port widens in a proximal direction. Additionally, the tapered configuration of the second pair of sidewalls 216 provides increased internal space for manipulation of the surgical instrument(s) "I" (FIG. 2), and acts to distribute the load applied to the patient during insertion and manipulation of the surgical instrument(s), e.g. instrument "I" of FIG. 2, in order to reduce patient trauma during the procedure.

The distal portion 206 is integral with, or connected to, the intermediate portion 208 and extends distally therefrom. The distal portion 206 includes a substantially cylindrical configuration defining an opening 218 that extends therethrough. It should be appreciated, however, that the distal portion 206 may include other geometrical configurations, e.g., substantially rectangular, substantially oval, etc. in alternative embodiments of the present disclosure. The opening 218 is configured and dimensioned to facilitate passage of the surgical instrument(s), e.g. instrument "I" of FIG. 2, through the patient's tissue and into the internal work site as it communicates with the opening in the proximal and intermediate portions 204, 208, thereby providing a passageway through the access port 200.

With continued reference to FIG. 3, an outer peripheral surface 209 of the intermediate portion 208 of access port 200 and/or an outer peripheral surface 211 of the proximal portion 210 and/or an outer peripheral surface of the distal portion 206 of access port 200 may include a pressure feedback indicator or member 220 disposed thereon, either on a portion thereof or on the entire surface. More particularly, similar to access port 100, the outer peripheral surfaces 209, 211 of the intermediate and proximal portion 208, 210, respectively, of access port 200 are coated with a biocompatible, pressure sensitive paint 220, although it is envisioned that other pressure feedback indicators may be provided. Alternatively, the outer peripheral surfaces 209, 211 can be formed partially or entirely from a pressure sensitive material. The pressure sensitive paint, as will be described in greater detail below with reference to FIG. 10, provides visual feedback as to the location and/or relative amount of pressure applied to the respective outer peripheral surfaces 209, 211 of the intermediate and proximal portions 208, 210 of access port 200. For use with certain procedures, the distal portion 206 in addition or alternatively can be formed from a pressure sensitive material or the pressure sensitive material can be disposed on the entire region or a portion thereof.

FIG. 4 illustrates another embodiment of the presently disclosed access port, which is identified by the reference character 300. In contrast to the aforedescribed embodiments, which are formed primarily from a substantially rigid material, the access port 300, and each variation thereof discussed herein below, incorporates substantially compliant structure, either partially or wholly. The incorporation of compliant structure allows for reconfiguration of the access port 300 during insertion, removal, and manipulation of the surgical instrument(s), e.g. instrument "I" of FIG. 2. The ability of the access port 300 to be reconfigured during use maximizes the space available within the access port 300 for manipulation of the surgical instrument(s) and facilitates more precise conformity with the shape of the intercostal space, thereby restricting movement of the access port 300 during the course of the surgical procedure, and consequently, reducing any effect upon the patient's tissue that would otherwise result from such movement.

The access port 300 includes a body portion 302 that is preferably entirely formed from a compliant material, e.g., polyurethane (PU) foam. The incorporation of such a material allows the body portion 302 of the access port 300 in certain instances to better conform to the shape of the intercostal space defined between the patient's adjacent ribs "R" and to minimize the force applied to the patient's tissue during insertion and removal of the access port 300. An opening/passageway extends through the access port 300 to receive surgical instruments therethrough.

The body portion 302 may be formed from a compliant material, e.g., foam 320, that exhibits pressure-sensitive characteristics. In other words, since the outer peripheral surface 303 of body portion 302 of access port 300 is formed from a pressure sensitive material 320, the pressure feedback indicator 320 is "built-in" to the outer peripheral surface 303 of access port 300. The functionality of the pressure feedback indicator 320, e.g., pressure sensitive foam 320, will be discussed in greater detail below with reference to FIG. 10. It is also contemplated that a pressure feedback indicator can be in the form of a coating or other material applied to the outer peripheral surface 303, either to a portion or to the entire surface thereof.

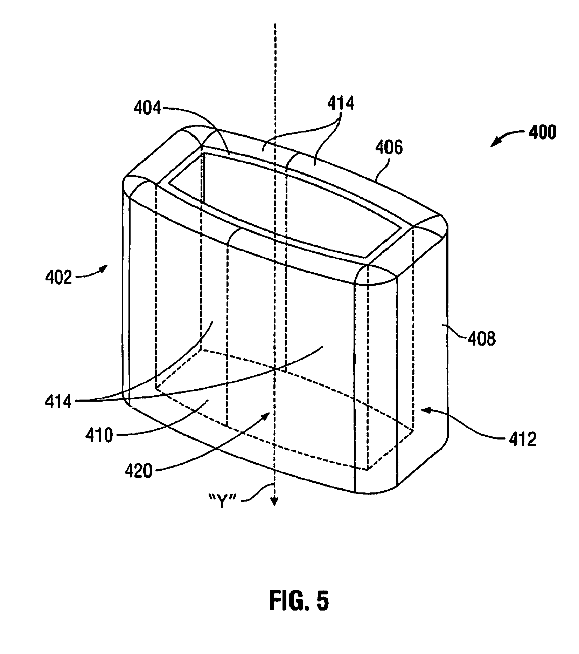

FIG. 5 illustrates another embodiment of the presently disclosed access port, which is identified by the reference character 400. The access port 400 includes a body portion 402 that extends along a longitudinal axis "Y" with a first liner 404 and a second liner 406 that is positioned thereabout. Whereas the first inner liner 404 is formed from a substantially rigid material, the second outer liner 406 is formed from a substantially compliant material, i.e., a material having a lower durometer than the material comprising the first liner 404, either partially or wholly. Consequently, during use of the access port 400, the compliant second liner 406 provides a cushioned contact area between the body portion 402 and the patient's tissue, e.g., the patient's ribs "R" shown in FIGS. 1 and 2. Specifically, the patient's ribs, and the surrounding tissue, can deform the second liner 406 inwardly towards the longitudinal axis "Y."

Access port 400 includes an opening/passageway extending therethrough (from a proximal to a distal portion) to receive surgical instruments.

The second liner 406 may be formed from a relatively thin, transparent material to define a pocket 412 between the second liner 406 and the first liner 404. Pocket 412 may divided into a plurality of compartments 414 disposed about the first liner 404 of access port 400. One or more of the compartments 414 of the pocket 412 may be filled either partially or entirely with a pressure feedback indicator 420, such as a pressure sensitive gel 420 configured for providing visual feedback as to the location and/or relative amount of pressure applied to the outer peripheral surface of first liner 404 and/or the outer peripheral surface of second liner 406 of access port 400. The liner(s) can be formed in whole or part of transparent material for visualization therethrough of the pressure sensitive material. The functionality of the pressure feedback indicator or member 420, e.g., pressure sensitive gel 420, will be discussed in greater detail below with reference to FIG. 10.

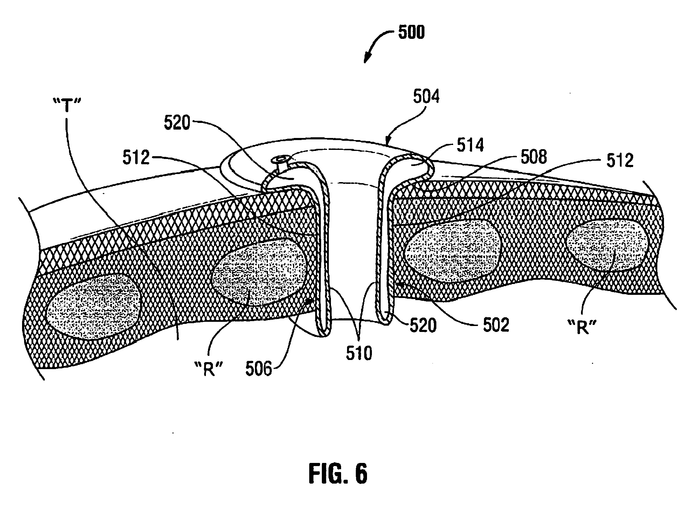

With reference now to FIG. 6, another embodiment of the presently disclosed access port, which is identified by the reference character 500, will be discussed. The access port 500, shown in cross-section, includes a body portion 502 having a proximal portion 504 and a distal portion 506. As shown in FIG. 6, the proximal portion 504 defines a width that is larger than the width defined by the distal portion 506, whereby the proximal portion 504 defines a flange, or buffer, 508. The flange 508 is configured and dimensioned for engagement with the patient's tissue upon positioning of the access port 500 within the intercostal space, as shown in FIG. 6, in order to prevent the access port 500 from passing entirely into the thoracic cavity "T." In one embodiment, it is envisioned that the access port 500 may further include a distal flange (not shown) to facilitate engagement with a distal surface of the patient's tissue, e.g., in order to further enhance stability of the access port 500.

The body portion 502 includes an inner membrane 510 and an outer membrane 512 which collectively define a cavity 514 therebetween. The respective inner and outer membranes 510, 512 may be formed from any suitable biocompatible material that is capable of retaining a fluid, e.g., air, water, or saline, within the cavity 514, i.e., a substantially impermeable material. During use of the access port 500, the cavity 514 is filled with the aforementioned fluid in order to transition the access port 500 between a deflated or collapsed condition and an inflated or expanded condition.

Inflating the access port 500 provides a measure of resiliency and deformability that cushions the patient's tissue during the course of the surgical procedure, and evenly distributes any applied forces, e.g., during insertion, removal, and/or manipulation of the surgical instrument(s), e.g. instrument "I" of FIG. 2, through the passageway/opening extending through access port 500 from proximal portion 504 to distal portion 506. Additionally, the inflatability of the access port 500 allows the access port 500 to more precisely conform to the shape of the intercostal space. Conformity with the specific configuration and dimensions of the intercostal space can minimize the force necessary to securely position the access port 500.

The access port 500 can be substantially circular or substantially oval in transverse cross-section, although other cross-sectional configurations are also contemplated.

With continued reference to FIG. 6, as in the previous embodiments, access port 500 may include a pressure feedback indicator or member 520. More particularly, the cavity 514 may be filled either partially or entirely with a pressure sensitive fluid, e.g., a pressure sensitive gel. In such an embodiment, the outer membrane 512 may be formed in whole or part from a transparent material to permit visualization therethrough (i.e., such that the pressure feedback mechanism 520 is visible through the outer membrane 512). Alternatively, the entire region or a portion thereof of the outer peripheral surface of access port 500 may be coated with a pressure sensitive paint, or access port 500 may be formed in whole or part from a pressure sensitive material. The functionality of the pressure feedback indicator 520, e.g., the pressure sensitive gel, will be discussed in greater detail below with reference to FIG. 10.

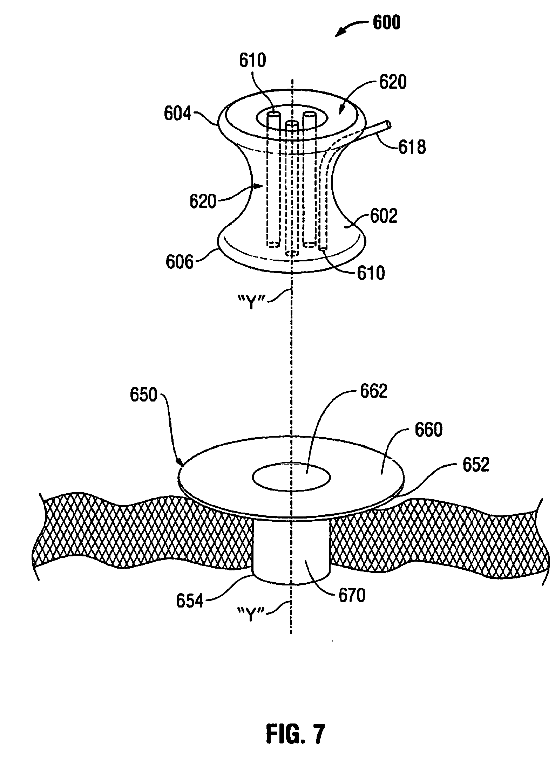

Referring now to FIG. 7, another embodiment of the access port of the present disclosure is shown as access portal 600. FIG. 7 illustrates an introducer assembly including an access portal 600 and an introducer 650.

Access portal 600 and introducer 650 are adapted for insertion within tissue, e.g., through the abdominal or peritoneal lining in connection with a laparoscopic surgical procedure to create an opening therethrough. Use in other procedures is also contemplated. In particular, when inserted within tissue, introducer 650 is adapted to establish a substantial seal with the tissue surfaces defining the opening in tissue. The introducer 650 will be described in greater detail hereinbelow.

With continued reference to FIG. 7, access portal 600 includes a body portion 602 having at least one longitudinal port 610 extending therethrough. The longitudinal ports 610 are generally parallel to the longitudinal axis "Y" of the access portal 600. The longitudinal ports 610 are dimensioned to receive a surgical object therethrough, e.g. a surgical instrument "I" as in FIG. 2. Access portal 600 is adapted to be enclosed within introducer 650. Upon introduction of an instrument or surgical object "I" (FIG. 2) through a respective port 610, the inner surface portions defining the port 610 establish and maintain a substantial sealed relation about the instrument or surgical object. Access portal 600 may define an hourglass shape as shown. Proximal and distal ends 604, 606, respectively, may define flange segments, which may be integrally formed with access portal 600. Access portal 600 may be made from a disposable, compressible, and/or flexible type material, for example, but not limited to, a suitable foam, gel material, or soft rubber having sufficient compliance to form a seal about one or more surgical objects, and also establish a sealing relation with tissue and/or the introducer 650. The foam is preferably sufficiently compliant to accommodate off axis motion of the surgical object.

Introducer 650 is adapted to facilitate insertion of access portal 600 through an opening in tissue. Introducer 650 is substantially elongated having a proximal end 652 and a distal end 654 defining a longitudinal axis "Y." Introducer 650 includes a surface indicator 660 and a portal indicator 670 extending therefrom. In the illustrated embodiments, surface member 660 is disk shaped, although other shapes are contemplated. Introducer 650 has a longitudinal channel 662 extending through surface indicator 660 and portal indicator 670 for reception and passage of access portal 600. Introducer 650 may be made of any type of suitable rigid material, for example, including but not limited to, metals and/or polymers.

Access portal 600 may be compressed to a compressed condition to permit at least partial passage through (insertion or extraction) the longitudinal channel 662 of the introducer 650. Once within the longitudinal channel 662, access portal 600 will return toward the normal expanded condition with an outer wall of the access portal 600 establishing a seal with the longitudinal channel 662. Access portal 600 may include an insufflation conduit 618 mounted within one of ports 610, or a separate port 618, and connectable to a source of insufflation fluid to permit passage of an insufflation fluid (e.g., CO₂), to create and/or maintain a working space in the pneumoperitoneum. The physician may insert various instruments through the access portal 600 and the introducer 650 for performing a procedure while maintaining a substantially sealed relationship with the surgical site.

As in the previous embodiments, access portal 600 may include a pressure feedback indicator or member 620, e.g., a coating of pressure sensitive paint or a pressure sensitive gel disposed over the entire outer region or disposed over a portion of the outer region, or access portal 600 may be formed in whole or in part from a pressure sensitive material, for providing visual feedback as to the location and/or relative amount of pressure applied to the access portal 600.

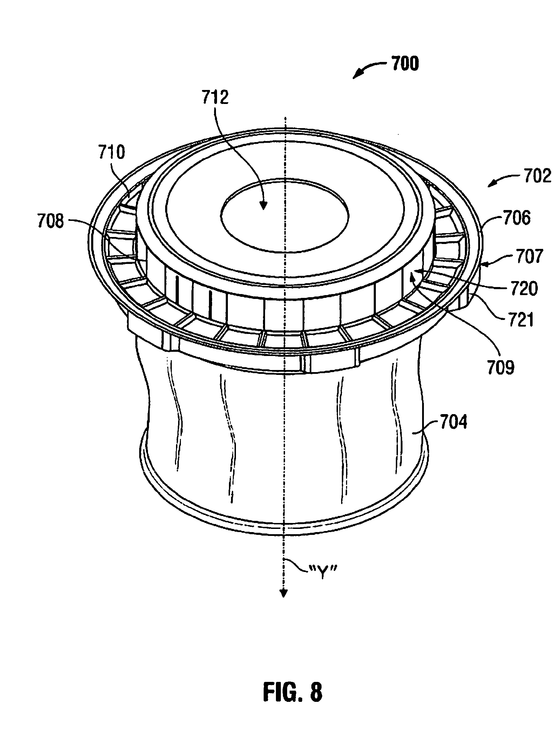

Turning now to FIGS. 8-9, yet another embodiment of the access port of the present disclosure is shown by reference numeral 700. Access port 700 includes an access housing, or body portion 702 defining longitudinal axis "Y" and a base 704 which extends distally from housing 702. Housing 702 includes outer base 706, ring 708 disposed within the outer base 706 and hub 710. Each of outer base 706, ring 708 and hub 710 are preferably substantially annular or ring-like in configuration defining a central aperture or passageway 712 to permit access through housing 702 and into the internal surgical site. Other configurations are also contemplated.

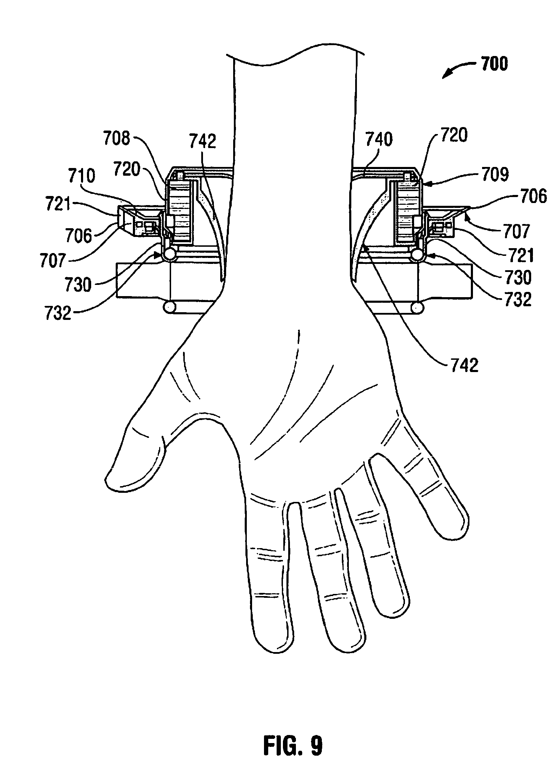

As shown in Fig. 9, access port 700 is configured to permit hand access to an internal surgical site. Thus, hand assisted surgery may be effected by advancement of the surgeon's hand and arm through seals 740, 742 of access housing 702 and into the surgical site. Seals 740, 742 form a fluid tight seal about the arm. The desired hand assisted procedure may then be performed within the sealed, internal surgical site.

An outer peripheral surface 709 of ring 708 of housing 702 may be coated in whole or in part with a pressure sensitive paint 720 (or other pressure feedback indicator) for providing visual feedback as to the location and/or relative amount of pressure applied to the access portal 700. Alternatively, ring 708 can be made in whole or in part of a pressure sensitive material.

Distal annular ring 730 may alternatively or additionally be coated with a pressure sensitive paint 732 (or other pressure feedback indicator) for providing visual feedback to the user as to the location and/or relative amount of pressure applied to the access portal 700. Distal annular ring 730 is positioned adjacent the incision in tissue and, thus, pressure sensitive paint 732 may also provide an indication as to the location and/or relative amount of pressure being applied to tissue surrounding the incision.

Alternatively, or additionally, an outer peripheral surface 707 of annular outer base 706 (and/or the outer peripheral surfaces of any of the other components of access port 700) may be coated with a pressure sensitive paint in whole or in part or be formed from a pressure sensitive material 721 (or other pressure feedback indicator) in whole or in part, for providing visual feedback as to the pressure on access port 700.

Turning now to FIG. 10, surgical access port 800 is shown positioned within an opening in tissue. Access port 800 is a schematic illustration showing the functionality of a pressure feedback indicator or member 820 used in conjunction with access port 800. The description hereinbelow of the function of access port 800 in conjunction with pressure feedback indicator 820 is shown by way of example. Accordingly, the following description applies similarly to the function of access ports 100-700 described above in conjunction with the respective pressure feedback indicators (members) 120-720. Consequently for brevity, only indicator 820 is described in detail.

Note the pressure feedback indicator 820 can also be used in conjunction with other access ports.

Access port 800 generally includes a body portion 810 defining a passageway 812 therethrough and has an outer peripheral surface 811. Pressure feedback indicator 820 is disposed on outer peripheral surface 811 of access port 800 and may include a bio-compatible pressure sensitive paint (or coating) coated on the entire outer peripheral surface 811 of access port 800 or on a portion thereof. Examples of such pressure sensitive paint include acrylate pressure sensitive coatings and polystyrene-polyisoprene pressure sensitive coatings. Alternatively, pressure feedback indicator 820 may include an encapsulated, pressure sensitive gel disposed about the outer peripheral surface 811 of access port 800 such as, for example, pressure sensitive silicon gels, pressure sensitive polyurethane gels and pressure sensitive polyacrylate gels. Alternatively, pressure feedback indicator 820 may be "built-in" to access port 800 itself. In other words, the outer peripheral surface 811 of access port 800 may be formed in whole or in part from a biocompatible pressure sensitive material made at least partially from, for example, polymers of polyurethane, polyacrylate and/or silicon.

As mentioned above, the pressure feedback indicator, e.g., pressure feedback indicator 820, is configured to provide a visual indication, e.g., a color change, at the location(s) on the outer peripheral surface 811 of access port 800 where the applied pressure is greater than a predetermined threshold. That is, the pressure feedback indicator may undergo a visible color change at the specific location on the outer peripheral surface 811 of the access port 800 where the applied pressure exceeds the predetermined threshold. Thus, access port 800 may exhibit an initial, or first color wherein the applied pressure is less than the predetermined threshold and may exhibit a second, or "indication" color when, at the location (or locations) of applied pressure, the applied pressure is greater than the pre-determined threshold. Additional indication colors or color shades could be provided to signal different pressures. That is, different colors or color shades can correspond to different pressure values.

The predetermined threshold may be determined by the specific characteristics of the pressure feedback indicator 820. Accordingly, the pressure feedback indicator 820 may be configured according to a specific predetermined threshold pressure which, in turn, may depend on the procedures being performed using the access port 800, the anatomical dimensions in which the access port 800 is to be used and/or the specific characteristics of access port 800.

As shown in FIG. 10, by way of example, when the pressure on access port 800 is below the predetermined threshold, the pressure feedback indicator 820 of access port 800 exhibits an initial, or first color "C₁." However, when the surgical instrument "I" is moved off the longitudinal axis "Y" in the direction of arrows "X," to contact (and thus pressure) the pressure feedback indicator 820 of access port 800, the pressured area, if the applied pressure is greater than a predetermined threshold, exhibits a second, or "indication" color "C₂." Additionally, the movement of surgical instrument "I" may urge access port 800 in the direction of arrows "X," especially where access port 800 is formed from a relatively rigid material, such that access port 800 is urged into surrounding tissue. If the surrounding tissue, e.g., tissue area "A," exerts a pressure on the access port 800 that is greater than the predetermined threshold, the pressure feedback indicator 820 of access port 800 may exhibit color "C₃" at the location of the applied pressure. Thus, the pressure feedback indicator, e.g. pressure sensitive material, can detect excess pressure of the instrument with respect to the access port and/or excess pressure of the tissue with respect to the access port.

Colors "C₂" and "C₃" may be the same, or a similar color, e.g., pressure feedback indicator 820 may be configured to exhibit two colors, an initial color "C₁" and an "indication" color "C₂," "C₃." Alternatively, colors "C₂" and "C₃" may be visibly different, i.e., pressure feedback indicator 820 may be configured to exhibit a range of colors, or shades of colors, depending on the relative amount of pressure applied at the specific location on the access port 800. That is, additional colors (or color shades) could be utilized for example to indicate increased pressures beyond the initial color changes of C₂, C₃.

As can be appreciated, providing a visual indication, e.g., a color change, at a specific location on the outer peripheral surface 811 of the access port 800 corresponding to an applied pressure above a predetermined threshold provides visual feedback to the surgeon as to the location (or locations) and/or relative amount(s) of such pressure. The surgeon may then use this information to modify, or alter the surgical procedure accordingly to prevent excess pressure on surrounding tissue and/or on the access port.

For example, with continued reference to FIG. 10, the color change "C₂" alerts the surgeon as to the location where significant pressure is applied to the access port 800. Even without noticing the color change "C₃," the surgeon would be alerted, due to the color change "C₂," to the fact that pressure is being applied to the access port 800 in the direction of arrows "X." Thus, the surgeon is alerted to the consequence of the pressure applied by instrument "I," namely that access port 800 may be urged into the surrounding tissue, e.g., area "A," in the direction of arrows "X." Accordingly, the surgeon may then adjust the positioning of the surgical instrument(s) "I" within the access port 800 and/or may modify the surgical technique used in order to alleviate the influence of such pressures upon the patient's tissues, and consequently, to reduce patient trauma, discomfort following the procedure, and recovery time.

The color change "C₃" further indicates to the surgeon the locations, both on the outer peripheral surface 811 of access port 800, and on the area "A" of surrounding tissue, wherein access port 800 is applying pressure to tissue above the predetermined threshold (and vice versa, i.e., wherein the surrounding tissue is pressuring access port 800 above the predetermined threshold). As described above, such a feature provides the surgeon with visual feedback, allowing the surgeon to modify, or alter the surgical procedure accordingly.

Further, it may be the case that, especially where the access port 800 is formed from a compliant material, that the pressure applied by instrument "I" and the pressure applied by the area of tissue "A," although both above the predetermined threshold, may be significantly different. Accordingly, the pressure feedback indicator 820 may provide for a spectrum of range of colors (or different colors) corresponding to specific pressures or to ranges of pressure above the predetermined threshold. Thus, pressure feedback indicator 820 may exhibit a darker color, or darker shade of color "C₂", where the applied pressure is greater, and a lighter color, or lighter shade of color "C₃", where the applied pressure is relatively less, but still above the threshold.

It is also contemplated that alternatively an audible signal could be provided to indicate to the user that a pressure exceeding the predetermined threshold has been applied to the access port. The audible signal could be provided such that it provides a spectrum of audible signals corresponding to specific pressures or to range of pressures above the predetermined threshold.

It should also be appreciated that visible signals of pressure changes other than color changes could be provided.

Persons skilled in the art will understand that the devices and methods specifically described herein and illustrated in the accompanying figures are non-limiting exemplary embodiments, and that the description, disclosure, and figures should be construed merely exemplary of particular embodiments. It is to be understood, therefore, that the present disclosure is not limited to the precise embodiments described, and that various other changes and modifications may be effected by one skilled in the art without departing from the scope or spirit of the disclosure. Additionally, it is envisioned that the elements and features illustrated or described in connection with one exemplary embodiment may be combined with the elements and features of another without departing from the scope of the present disclosure, and that such modifications and variations are also intended to be included within the scope of the present disclosure. Accordingly, the subject matter of the present disclosure is not to be limited by what has been particularly shown and described, and those skilled in the art will envision other modifications within the scope and spirit of the present disclosure.

### The invention may be described by reference to the following numbered paragraphs:

1. A surgical access port configured and dimensioned for positioning within an opening in tissue for providing access to an internal surgical site, the surgical access port comprising a body portion defining a passageway therethrough and including an outer surface; and a pressure feedback indicator on at least a portion of the outer surface of the body portion, the pressure feedback indicator configured for providing a visual indication to a user where an applied pressure to the body portion is greater than a predetermined threshold.
2. The surgical access port according to paragraph 1, wherein the pressure feedback indicator includes a pressure sensitive paint coating at least a portion of the outer surface.
3. The surgical access port according to paragraph 1, wherein the pressure feedback indicator includes a pressure sensitive gel encapsulated in a casing and disposed about at least a portion of the outer surface.
4. The surgical access port according to paragraph 1, wherein the pressure feedback indicator includes a pressure sensitive material that forms at least a portion of the outer surface.
5. The surgical access port according to paragraph 1, wherein the visual indication includes a change in color of the pressure feedback indicator at the location on the outer surface of the body portion where the applied pressure is greater than the predetermined threshold.
6. The surgical access port according to paragraph 1, wherein the pressure feedback indicator changes color at the location on the outer surface between an initial color, wherein the applied pressure at the location on the outer surface is less than the predetermined threshold, and an indication color, wherein the applied pressure at the location on the outer surface is greater than the predetermined threshold.
7. The surgical access port according to paragraph 1, wherein the pressure feedback indicator is further configured to provide a visual indication as to a relative amount of applied pressure on the outer surface.
8. The surgical access port according to paragraph 7, wherein the pressure feedback indicator changes color at the location on the outer surface through a spectrum of colors between an initial color, wherein the applied pressure is equal to or less than the predetermined threshold, and one of several other colors, depending on the amount the applied pressure is greater than the predetermined threshold.
9. The surgical access port according to paragraph 1, wherein the body portion of the access port has a first region external of the opening in tissue and a second region internal of the opening in tissue, wherein the pressure feedback indicator is on the first region of the body portion.
10. The surgical access port according to paragraph 1, wherein the body portion of the access port has a first region external of the opening in tissue and a second region internal of the opening in tissue, wherein the pressure feedback indicator is on the second region of the body portion.
11. The surgical access port according to paragraph 9, wherein the pressure feedback indicator is further on the second region of the body portion.
12. A surgical access port configured and dimensioned for positioning within an opening in tissue for providing access to an internal surgical site, the surgical access port comprising a body portion defining a passageway therethrough and having a proximal region and a distal region, the body portion extending through the opening in tissue and including a pressure feedback indicator, the pressure feedback indicator providing an indication to the user of a change in pressure on the body portion.
13. The surgical access port according to paragraph 12, wherein the pressure feedback indicator is at the proximal region of the body portion and is responsive to pressure applied by a medical instrument extending through the passageway of the body portion.
14. The surgical access port according to paragraph 12, wherein the pressure feedback indicator is at the distal region of the body portion and is responsive to pressure applied by the tissue on the body portion.
15. The surgical access port according to paragraph 13, wherein the pressure feedback indicator is further at the distal region of the body portion and is responsive to pressure applied by the tissue.
16. The surgical access port according to paragraph 12, wherein the pressure feedback indicator provides a color change in response to the change in pressure.
17. The surgical access port according to paragraph 16, wherein the indication includes a change to a first indication color in a first region and a change to a second indication color in a second region.
18. A method of facilitating access to an internal surgical site beneath a patient's tissue comprising the steps of forming an opening in the patient's tissue, advancing an access port through the opening, the access port defining a passageway therethrough and including a pressure feedback indicator on at least a portion of the outer surface thereof, the pressure feedback indicator configured for providing a visual indication as to the location on the outer surface where an applied pressure is greater than a pre-determined threshold, beginning a surgical procedure within the internal surgical site via the passageway defined within the access port, observing the visual indication as to the location on the outer surface where the applied pressure is greater than the predetermined threshold and modifying the surgical procedure according to the observed visual indication to reduce the applied pressure at the location on the outer surface to a pressure less than the predetermined threshold.
19. The method according to paragraph 18, wherein the visual indication includes a change in color of the pressure feedback indicator at the location on the outer surface where the applied pressure is greater than the predetermined threshold.
20. The method according to paragraph 18, wherein the pressure feedback indicator is further configured to provide a visual indication as to a relative amount of applied pressure at the location on the outer surface.

## Claims

1. A surgical access port configured and dimensioned for positioning within an opening in tissue for providing access to an internal surgical site, the surgical access port comprising a body portion defining a passageway therethrough and having a proximal region and a distal region, the body portion extending through the opening in tissue and including a pressure feedback indicator, the pressure feedback indicator providing an indication to the user of a change in pressure on the body portion.

2. The surgical access port according to claim 1, wherein the pressure feedback indicator is configured for providing a visual indication to a user where an applied pressure to the body portion is greater than a predetermined threshold.

3. The surgical access port according to claims 1 or 2, wherein the pressure feedback indicator includes a pressure sensitive paint coating at least a portion of the outer surface.

4. The surgical access port according to claims 1 or 2, wherein the pressure feedback indicator includes a pressure sensitive gel encapsulated in a casing and disposed about at least a portion of the outer surface.

5. The surgical access port according to claims 1 or 2, wherein the pressure feedback indicator includes a pressure sensitive material that forms at least a portion of the outer surface.

6. The surgical access port according to any of claims 2-5, wherein the visual indication includes a change in color of the pressure feedback indicator at the location on the outer surface of the body portion where the applied pressure is greater than the predetermined threshold.

7. The surgical access port according to any of claims 2-6, wherein the pressure feedback indicator changes color at the location on the outer surface between an initial color, wherein the applied pressure at the location on the outer surface is less than the predetermined threshold, and an indication color, wherein the applied pressure at the location on the outer surface is greater than the predetermined threshold.

8. The surgical access port according to any of claims 1-7, wherein the pressure feedback indicator is further configured to provide a visual indication as to a relative amount of applied pressure on the outer surface.

9. The surgical access port according to claim 8, wherein the pressure feedback indicator changes color at the location on the outer surface through a spectrum of colors between an initial color, wherein the applied pressure is equal to or less than the predetermined threshold, and one of several other colors, depending on the amount the applied pressure is greater than the predetermined threshold.

10. The surgical access port according to any of claims 1-9, wherein the body portion of the access port has a first region external of the opening in tissue and a second region internal of the opening in tissue, wherein the pressure feedback indicator is on the first region of the body portion.

11. The surgical access port according to any of claims 1-10, wherein the body portion of the access port has a first region external of the opening in tissue and a second region internal of the opening in tissue, wherein the pressure feedback indicator is on the second region of the body portion.

12. The surgical access port according to any of claims 1-11, wherein the pressure feedback indicator is at the proximal region of the body portion and is responsive to pressure applied by a medical instrument extending through the passageway of the body portion.

13. The surgical access port according to any of claims 1-12, wherein the pressure feedback indicator is at the distal region of the body portion and is responsive to pressure applied by the tissue on the body portion.

14. The surgical access port according to any of claims 1-13, wherein the pressure feedback indicator provides a color change in response to the change in pressure.

15. The surgical access port according to any of claims 1-14, wherein the indication includes a change to a first indication color in a first region and a change to a second indication color in a second region.
